(19) Europäisches Patentamt
European Patent Office
Office européen des brevets

(11) Numéro de publication: **0 110 746 B1**

# (12) FASCICULE DE BREVET EUROPEEN

(45) Date de publication du fascicule du brevet: **11.06.86**

(51) Int. Cl.⁴: **C 07 C 93/06**, A 61 K 31/13

(21) Numéro de dépôt: **83402068.7**

(22) Date de dépôt: **25.10.83**

(54) **Dérivés de 3-amino-1-phénoxy-2-propanol, utilisation en thérapeutique et procédé de préparation.**

(30) Priorité: **26.10.82 FR 8217934**

(43) Date de publication de la demande: **13.06.84 Bulletin 84/24**

(45) Mention de la délivrance du brevet: **11.06.86 Bulletin 86/24**

(84) Etats contractants désignés: **AT BE CH DE FR GB IT LI LU NL SE**

(56) Documents cités: **FR - M - 4 061**

(73) Titulaire: **LABORATOIRE L. LAFON Société anonyme dite:, 1 rue Georges Médéric, F-94701 Maisons-Alfort (FR)**

(72) Inventeur: **Lafon, Louis, 5 rue de l'Alboni, F-75016 Paris (FR)**

(74) Mandataire: **Combe, André, CABINET BEAU DE LOMENIE 55 rue d'Amsterdam, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 110 746 B1

LIBER, STOCKHOLM 1986

**Description**

La présente invention concerne en tant que produits industriels nouveaux des dérivés de 3-amino-1-phénoxy-2-propanol à savoir les 3-alkylamino-1-(2-chloro-3 5-diméthoxyphénoxy)-2-propanols de formule (1) ci-après et leurs sels. Elle concerne également l'utilisation en thérapeutique et le procédé de préparation de ces nouveaux produits.

On sait notamment des brevets français n° 3847M n° 4061M et n° 70.13806 et du brevet américain n° 3 203 992, que, d'une manière générale les dérivés appartenant à la famille des 3-amino-1-phénoxy-2-propanols ont des propriétés β-bloquantes. Or il se trouve que les dérivés de ladite famille n'ont jamais pu être commercialisés en raison d'un certain nombre d'inconvénients notamment des difficultés de tolérance et/ou des durées d'effet β-bloquant trop courtes.

On vient de trouver de façon surprenante que les 3-alkylamino-1-(2-chloro-3 5-diméthoxyphénoxy)-2-propanols selon l'invention ne présentent pas les inconvénients susvisés, sont sur le plsn thérapeutique plus efficaces que (i) le 1-(3,5-diméthoxyphénoxy)-3-isopropylamino-2-propanol des BSM n° 3647M et n° 4081M, et (ii)le 1-(3 5-diméthoxyphénoxy)-3-tertiobutylamino-2-propanol du brevet français n° 70.13808, et sont au moins aussi intéressants que le propanolol un produit β-bloquant de référence qui est structurellement différent.

Les nouveaux dérivés de 3-amino-1-phénoxy-2-propanol selon l'invention sont caractérisés en ce qu'ils sont choisis parmi l'ensemble constitué par

(i) les 3-alkylamino-1-(2-chloro-3,5-diméthoxyphénoxy)-2-propanols répondant à la formule générale

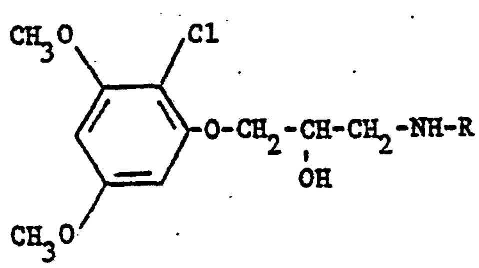

(I)

dans laquelle R est $CH(CH_3)_2$ ou $C(CH_3)_3$; et

(ii) leurs sels d'addition.

D'un point de vue pratique, les produits préférés selon l'invention sont le 1-(2-chloro-3,5-diméthoxyphénoxy)-3-tertiobutyl-amino-2-propanol et ses sels d'addition, notamment le chlorhydrate. Les essais chez l'animal et chez l'homme ont montré que le dérivé 3-tertiobutylamino et ses sels sont plus actifs que le dérivé 3-isopropylamino et ses sels.

Par sels d'addition, on entend ici les sels d'addition d'acides obtenus par réaction d'une base libre de formule (1) avec un acide minéral ou organique, et les sels d'ammonium. Parmi les acides utilisables pour salifier les bases de formule (1), on peut notamment citer les acides chlorhydrique, bromhydrique, nitrique, sulfurique, formique, acétique, propionique, oxalique, fumarique, maléique, succinique, benzoique, cinnamique, mandélique, citrique, malique, tartrique, aspartique, glutamique, p-toluènesulfonique et méthanesulfonique. Parmi les composés permettant d'obtenir des sels d'ammonium, on peut citer notamment $ICH_3$ et $ClCH_3$. Les sels d'addition d'acides, notamment les chlorhydrates, sont les sels d'addition préférés en thérapeutique.

Les nouveaux dérivés de 3-amino-1-phénoxy-2-propanol selon l'invention peuvent être préparés selon une méthode connue en soi par application de mécanismes réactionnels classiques. Le procédé que l'on préconise comprend deux étapes, à savoir

a) la réaction du 2-chloro-3 5-diméthoxyphénol de formule

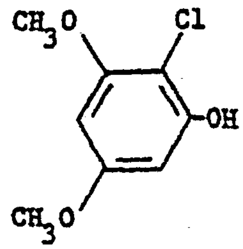

(II)

avec l'épichlorhydrine III en présence de pyridine, pour obtenir le 1-(2-chloro-3,5-diméthoxyphénoxy)-2 3-époxypropane de formule

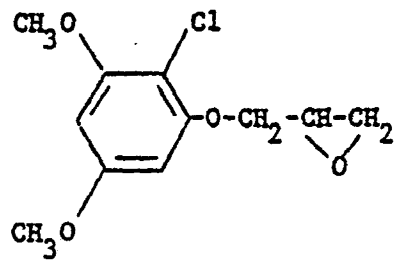

(IV)

puis,

b) la réaction de l'époxyde IV avec une amine

$NH_2R$ (V)

(où R est i-$C_3H_7$ ou t-$C_4H_9$) dans un alcool inférieur, notamment un alcool en $C_1$-$C_3$, de préférence l'éthanol ou le méthanol.

Le meilleur mode de mise en oeuvre consiste lors de l'étape a), à faire réagir 1 mole de II avec 4 à 10 moles de III pendant 2 à 5 h à une température comprise entre 100 et 120°C l'épichlorhydrine III servant de solvant et la pyridine de catalyseur et, lors de l'étape b) à faire réagir 1 mole d'époxyde IV avec 8 à 12 moles d'amine V en présence d'éthanol, à la température de reflux du milieu réactionnel pendant au moins 0,5 h.

Un certain nombre de composés selon l'invention a été consigné de façon non limitative dans le tableau 1 ci-après.

**TABLEAU 1**

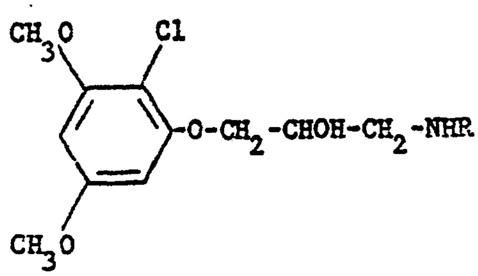

| Produit | N° de code | R | Point de fusion |
|---|---|---|---|
| Exemple 1(b) | CRL 41058 | $t\text{-}C_4H_9$ | 216°C |
| Exemple 2(a) | — | $t\text{-}C_4H_9$ | 85°C |
| Exemple 3(b) | CRL 41045 | $i\text{-}C_3H_7$ | 206°C |
| Exemple 4(a) | — | $i\text{-}C_3H_7$ | 97°C |

Notes :
(a) : base libre
(b) : chlorhydrate

Les 3-alkylamino-1-(2-chloro-3 5-diméthoxyphénoxy)-2-propanols de formule (1) et leurs sels d'addition sont utiles en tant que médicaments β-bloquants. On préconise selon l'invention une composition thérapeutique qui est caractérisée en ce qu'elle renferme, en association avec un excipient physiologiquement acceptable, au moins un composé de formule (1) ci-dessus ou l'un de ses sels d'addition non toxiques en tant qu'agent β-bloquant.

Par ailleurs, les composés selon l'invention, et notamment les bases libres, interviennent comme intermédiaires de synthèse dans la préparation d'autres substances comme indiqué dans la demande de brevet français n° 82-17935 déposée par la demanderesse le même jour que la présente invention.

D'autres avantages et caractéristiques de l'invention seront mieux compris à la lecture qui va suivre d'exemples de préparation nullement limitatifs mais donnés à titre d'illustration.

**PREPARATION 1**

**Obtention du chlorhydrate de 1-(2-chloro-3,5-diméthoxyphénoxy)-3-tertiobutylamino-2-propanol.**
(Exemple 1; n° de code CRL 41 058)

**1°) 2-Chloro-3 5-diméthoxyphénol**

Dans une solution maintenue à une température comprise entre 35 et 40°C et constituée par 75 g (0,487 mole) de 3,5-diméthoxy-phénol et 400 ml de $CCl_4$, on introduit par fractions, en 2,5 h, une quantité de 70,5 g (0,528 mole) de N-chlorosuccinimide. On chauffe ensuite pendant 0 25 h à une température de 70°C environ. On élimine le précipité formé par filtration et amène le filtrat à siccité sous pression réduite. Le résidu d'évaporation ainsi obtenu est purifié par distillation sous vide puis lavage du distillat avec de l'eau. On obtient 56 5 g (rendement : 61 65%) de 2-chloro-3,5-diméthoxy-phénol. $F_{inst}$ (Köfler) = 57°C.

**2°) 1-(2-chloro-3 5-diméthoxyphénoxy)-2,3-époxyeropane**

On chauffe vers 115°C pendant 4 h une solution de 40 g (0,212 mole) de 2-chloro-3,5-diméthoxyphénol dans 133 ml (1,697 mole) d'épichlorhydrine en présence de 0,25 ml de pyridine. On amène le milieu réactionnel à siccité, on reprend le résidu par de l'acétate d'éthyle et on lave successivement la phase acétate d'éthyle par de la soude et de l'eau. Après séchage sur sulfate de sodium anhydre et évaporation du solvant, on obtient une huile brune épaisse. Cette huile est purifiée par cristallisation dans l'isopropanol pour donner 36,5 g (rendement :

70,4%) de 1-(2-chloro-3,5-diméthoxyphénoxy)-2,3-époxypropane qui se présente sous la forme d'une poudre de couleur marron-chocolat. $F_{inst}$ (Köfler) = 96°C.

**3°) CRL 41 058**

On chauffe au reflux pendant 1 h une solution de 36 g (0,147 mole) de 1-(2-chloro-3,5-diméthoxyphénoxy)-2,3-époxypropane et de 107,5 g (1,472 mole) de tertiobutylamine dans 150 ml d'éthanol anhydre. On amene le milieu réactionnel à siccité et on purifie le résidu ainsi obtenu par cristallisation dans l'éther diisopropylique pour obtenir 37 g de 1-(2-chloro-3,5-diméthoxyphénoxy)-3-tertiobutyl-amino-2-propanol qui se présente sous la forme d'une poudre beige. $F_{inst}$ (Köfler) = 85°C.

La base libre ainsi obtenue est traitée au reflux dans de l'éthanol anhydre par de l'éthanol chlorhydrique. Il se forme un précipité que l'on isole pour recueillir 36,3 g (rendement = 69,75%) de CRL 41 058 qui se présente sous la forme d'une poudre légèrement beige soluble à 12,5 g/l dans l'eau. $F_{inst}$ (Köfler) = 216°C.

**PREPARATION II**

**Obtention du chlorhydrate de 1-(2-chloro-3,5-diméthoxyphénoxy)-3-isopropylamino-2-propanol.**
(Exemple 3; n° de code CRL 41 045)

En procédant comme indiqué au stade 3°) de la préparation I, mais en remplaçant la tertiobutylamino par l'isopropylamine, on obtient le CRL 41 045. $F_{inst}$ (Köfler) = 206°C.

On a résumé ci-après une partie des essais qui ont été entrepris avec le CRL 41 058 (produit de l'exemple 1). Dans ces essais et sauf indications contraires, le CRL 41 058, en suspension dans une solution aqueuse de gomme arabique pour des concentrations supérieures ou égales à 3 g/l, et en solution dans de l'eau distillée pour des concentrations inférieures à 3 g/l, a été administré par voie intrapéritonéale sous un volume de 20 ml/kg chez la souris mâle et de 5 ml/kg chez le rat mâle.

**TOXICITE**

Chez la souris male par voie I.p. la DL-0 (dose maximale non mortelle) est supérieure à 64 mg/kg, et la DL-30 (dose à laquelle 30% des animaux meurent) est de l'ordre de 128 mg/kg. Le CRL 41 058 est moins toxique que le chlorhydrate de 1-(3,5-diméthoxy-phénoxy)-3-tertiobutylamino-2-propanol qui a une DL-50 par voie I.P. chez la souris mâle de l'ordre de 73 mg/kg.

**COMPORTEMENT GLOBAL ET REACTIVITES**

Des lots de 3 animaux sont observés avant, puis 15 min, 30 min, 1 h, 2 h, 3 h et 24 h après administration du CRL 41 058 par voie I.P. On constate que

1°) <u>chez la souris</u>

- les doses de 0,5 mg/kg, 2 mg/kg et 8 mg/kg ne provoquent pas de symptômes nets;
- la dose de 32 mg/kg conduit à une sédation inconstante (chez 2 animaux sur 3) et fugace (30 min), une dyspnée (chez 2 animaux sur 3) pendant 15 à 30 min; et

2°) <u>chez le rat</u>

- les doses de 0,25 mg/kg, 1 mg/kg et 4 mg/kg ne provoquent pas de symptômes nets;
- la dose de 16 mg/kg donne une sédation fugace (30 min) et déprime la respiration (chez 2 animaux sur 3) pendant 30 min.

**ETUDE DES PROPRIETES CARDIOVASCULAIRES**

**A - CHIEN ANESTHESIE**

Trois chiens (poids moyen : 13,6 kg) anesthésiés au nembutal reçoivent le CRL 41 058 par voie intraduodénale aux doses successives de 0,5 mg/kg, 1 mg/kg, 2 5 mg/kg, 5 mg/kg et 10 mg/kg. On mesure la pression artérielle (il est rappelé que 1 mm Hg correspond à environ 1,333 x $10^2$ Pa), la fréquence cardiaque, le débit artériel fémoral, le débit artériel vertébral, la température rectale et on note la coloration de la peau et la coloration de la bile recueillie par cathétérisation du cholédoque après ligature du cystique.

Le CRL 41 058 est bradycardisant dès la dose de 0 5 mg/kg sans aucune action nette sur la pression artérielle. 11 n'est pas vasodilatateur vertébral. L'augmentation du débit fémoral est présente seulement chez un chien sur trois dont le débit témoin est faible (24 ml/min). Chez les deux autres chiens, le débit diminue. Les températures rectale et cutanée varient peu. La coloration de la peau et celle de la bile ne sont pas modifiées.

Les effets de l'isoprénaline testés après la dose cumulée de 19 mg/kg de CRL 41 058 sont fortement diminués:

3 µg/kg d'isoprénaline font passer la pression artérielle diastolique à 119 mm Hg au lieu de 48 mm Hg et la fréquence cardiaque à 110 bat/min au lieu de 245 bat/min en témoin. Chez deux chiens où la dose d'isoprénaline a été augmentée jusqu'à 100 µg/kg, l'effet tachycardisant à cette dose est inférieur à l'effet tachycardisant de 0,3µg/kg d'isoprénaline témoin.

L'hypertension à la noradrénaline est aussi diminuée: à la dose de 1 µg/kg de noradrénaline la pression artérielle systolique passe à 221 mm Hg au lieu de 280 mm Hg en témoin.

**B - OREILLETTES ISOLEES DE COBAYE**

5 oreillettes droites isolées de cobaye sont utilisées pour déterminer la $pA_2$ du CRL 41 058 vis-à-vis de l'effet chronotrope de l'isoprénaline; après 15 min de contact la moyenne des $pA_2$ est de 8,21 ± 0,272.

Sur ces oreillettes, on évalue aussi l'action inotrope et chronotrope propre du CRL 41 058; aucune moyenne n'a pu être faite les concentrations et l'ordre d'administration des concentrations étant variables. Le CRL 41 058 possède un effet ino+ et chrono+ qui est maximum à la première concentration utilisée quelle qu'elle soit (3 x $10^{-8}$ à $10^{-6}$ M). par contre si, par exemple la concentration de $10^{-6}$ M est administrée après des concentrations inférieures qui ont été stimulantes cette concentration de $10^{-6}$ M est ino− et chrono − (ce phénomène pourrait laisser supposerune libération de catécholamines).

**C - RAT AHESTHESIE**

Des rats normotendus sont anesthésiés au pentobarbital (75 mg/kg 1.p.) et atropinés (1 mg/kg 1.F.) On mesure leur pression artérielle carotidienne, leur fréquence cardiaque par intégration de la pression artérielle. Une veine jugulaire est cathétérisée pour les injections. Si besoin est, l'anesthésie est entretenue par injection sous-cutanée de pentobarbital.

**1) Détermination de la dose β-bloquante efficace**

3 rats reçoivent le CRL 41 058 par voie intraveineuse, aux doses successives de 0 3 mg/kg, 1 mg/kg, 3 mg/kg, 10 mg/kg et, éventuellement, 30 mg/kg. On détermine la dose qui bloque la tachycardie indufte par 0,03 à 0,3 µg/kg I.V. d'isoprénaline (dose nécessaire pour obtenir une augmentation de fréquence cardiaque de 30 à 50 bat/min). On trouve que la dose en question est de 1 mg/kg I.V.

**2) Durée de l'action β-bloquante**

5 rats reçoivent 1 mg/kg de CRL 41 058 par voie I.V. On observe que :

- la pression artérielle diastolique n'est pas modifiée elle reste voisine de 91 mm Hg; et
- la fréquence cardiaque passe en 5 min, de 345 à 290 bat/min (soit -16%), la bradycardie maximale est obtenue au temps 20 min : 280 bat/min (soit -19%). Après 90 min, la fréquence cardiaque est à 307 bat/min (soit -11%).

Par ailleurs, la dose de 0,03 µg/kk I.V. d'isoprénaline fait passer :

- la pression artérielle de 91 à 65 mm Hg (soit -26 mm Hg); après 1 mg/kg I.V. de CRL 41 058, la variation de pression artérielle est de +8 mm Hg (-69% par rapport à la variation témoin) à 5 min et pendant les 2 h d'observation; et
- la fréquence cardiaque de 345 à 384 bat/min (soit + 39 bat/min); la tachycardie totalement inhibée par le CRL 41 058 au temps 5 min reprend 50% de sa valeur au temps 20 min; elle est restaurée à 92% de sa valeur au temps 60 min.

Enfin, chez 5 rats témoins recevant du sérum physiologique à la place du produit, on observe que :

- la pression artérielle reste constante; l'hypotension à l'isoprénaline diminue de 40% au cours de l'essai; et
- la fréquence cardiaque diminue de 28 bat/min à la suite des injections de nembutal nécessaires au maintien de l'anesthésie; la tachycardie à l'isoprénaline reste constante pendant les 2 h d'observation.

**D - RAT EVEILLE**

6 rats génétiquement hypertendus à cathéter artériel fémoral implanté reçoivent le CRL 41 058 à la dose de 20 mg/kg V.B.

La pression artérielle passe de 191 à 167 mm Hg au temps 6 h (- 13%; variation statistiquement significative). La fréquence cardiaque diminue dès 5 min elle passe de 382 à 322 bat/min (- 15%; variation statistiquement significative); la bradycardie maximale est atteinte à 2 h (305 bat/min soit - 20%; variation statistiquement significative), elle diminue ensuite pour être, à 7 h, de 322 bat/min, soit - 15%; variation statistiquement significative).

24 h après administration du CRL 41 058, on observe que la pression artérielle a repris sa valeur témoin, la fréquence cardiaque étant encore 330 bat/min (soit - 13%; variation statistiquement significative).

**E - CONCLUSION**

Il résulte des essais résumés ci-dessus que le CRL 41 058 est un très bon agent β-bloquant.

Les essais cliniques ont confirmé chez l'homme l'intérêt de l'action β-bloquante du CRL 41 058 à la dose quotidienne de 40 à 50 mg (répartie en 2 à 3 prises) pendant 2 à 6 semaines.

**Revendications** pour les Etats contractants: BE, CH, DE, FR, GB, IT, LI, LU, NL, SE

1. Dérivé de 3-amino-1-phénoxy-2-propanol, caractérisé en ce qu'il est choisi parmi l'ensemble constitué par (i) les 3-alkylamino-1-(2-chloro-3,5-diméthoxyphénoxy)-2-propanols répondant à la formule générale

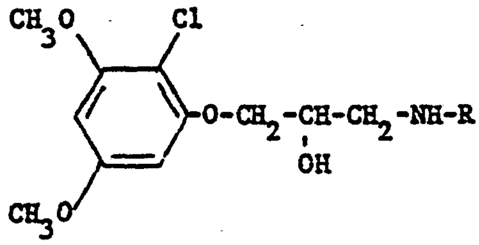

(I)

dans laquelle R est $CH(CH_3)_2$ ou $C(CH_3)_3$; et
(ii) leurs sels d'addition.

2. 1-(2-Chloro-3,5-diméthoxyphénoxy)-3-tertiobutylamino-2-propanol et ses sels d'addition.

3. 1-(2-Chloro-3,5-diméthoxyphénoxy)-3-isopropylamino-2-propanol et ses sels d'addition.

4. Composition thérapeutique, caractérisée en ce qu'elle renferme, en association avec un excipient physiologiquement acceptable, au moins un 3-alkylamino-1-(2-chloro-3,5-diméthoxyphénoxy)-2-propanol de formule (I) selon la revendication 1 ou l'un de ses sels d'addition non toxiques.

5. Procédé de préparation d'un composé de formule (I) selon la revendication 1, caractérisé en ce que
a) on fait réagir un 2-chloro-3 5-diméthoxyphénol de formule

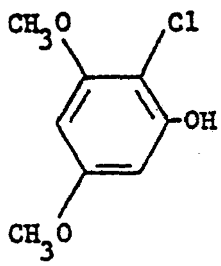

(II)

avec l'épichlorhydrine III, en présence de pyridine pour obtenir le 1-(2-chloro-3,5-diméthoxyphénoxy)-2,3-époxypropane de formule

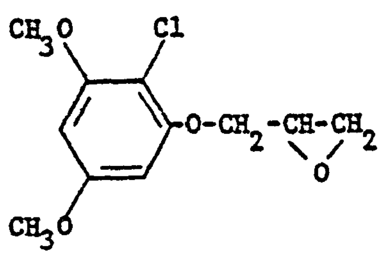

(IV)

puis
b) on fait réagir l'époxyde IV ainsi obtenu avec une amine
$HH_2R$ (V)
(où R est i-$C_3H_7$ ou t-$C_4H_9$) dans un alcool inférieur en $C_1$-$C_3$.

6. Procédé selon la revendication 5, caractérisé en ce que, lors de l'étape a), on fait réagir 1 mole de II avec 4 à 10 moles de III pendant 2 à 5 h à une température comprise entre 100 et 120°C;et en ce que lors de l'étape b) on fait réagir 1 mole d'époxyde IV avec 8 à 12 moles d'amine V en présence d'éthanol à la température de reflux du milieu réactionnel pendant au moins 0,5 h.

**Revendications** pour l'Etat contractant: AT

1. Utilisation d'un dérivé de 3-amino-1-phénoxy-2-propanol, choisi parmi l'ensemble constitué par : (i) les 3-alkylamino-1-(2-chloro-3,5-diméthoxyphénoxy)-2-propanols répondant à la formule générale

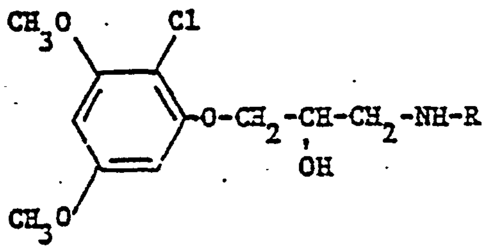

(I)

dans laquelle R est $CH(CH_3)_2$ ou $C(CH_3)_3$; et,
(ii) leurs sels d'addition non toxiques.
pour la fabrication d'un médicament.

2. Utilisation selon la revendication 1, caractérisée en ce que le composé est le 1-(2-chloro-3,5-diméthoxyphénoxy)-3-tertio-butylamino-2-propanol et ses sels d'addition.

3. Utilisation selon la revendication 1, caractérisée en ce que le composé est le 1-(2-chloro-3,5-diméthoxyphénoxy)-3-isopropyl-amino-2-propanol et ses sels d'addition.

4. Utilisation selon l'une des revendications 1 à 3, caractérisée en ce qu'au moins un dérivé de 3-amino-1-phénoxy-2-propanol ou l'un de ses sels d'addition non toxiques est en association avec un excipient physiologiquement acceptable.

5. Procédé de préparation d'un composé de formule (I) selon revendication 1, caractérisé en ce que
a) on fait réagir un 2-chloro-3 5-diméthoxyphénol de formule

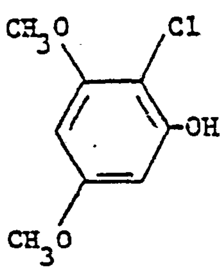

(II)

avec l'épichlorhydrine III en présence de pyridine pour obtenir le 1-(2-chloro-3 5-diméthoxyphénoxy)-2,3-époxypropane de formule

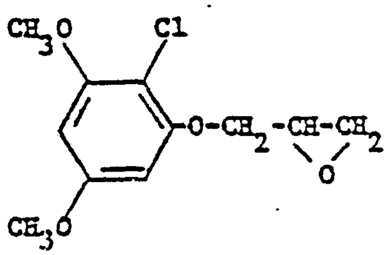

(IV)

puis
b) on fait réagir l'époxyde IV ainsi obtenu avec une amine
$NH_2R$ (V)
(où R est i-$C_3H_7$ ou t-$C_4H_9$) dans un alcool inférieur en $C_1$-$C_3$.

6. Procédé selon la revendication 5 caractérisé en ce que lors de l'étape a) on fait réagir 1 mole de II avec 4 à 10

moles de III pendant 2 à 5 h à une température comprise entre 100 et 120°C;et en ce que lors de l'étape b) on fait réagir 1 mole d'époxyde IV avec 8 à 12 moles d'amine V en présence d'éthanol à la température de reflux du milieu réactionnel pendant au moins 0,5 h.

**Patentansprüche** für die Vertragsstaaten BE, CH, DE, FR, GB, IT, LI, LU, NL, SE

1. 3-Amino-1-phenoxy-2-propanol-Derivat, dadurch gekennzeichnet, daß es ausgewählt ist aus der Gruppe bestehend aus
(i) den 3-Alkylamino-1-(2-chlor-3,5-dimethoxyphenoxy)-2-propanolen der allgemeinen Formel

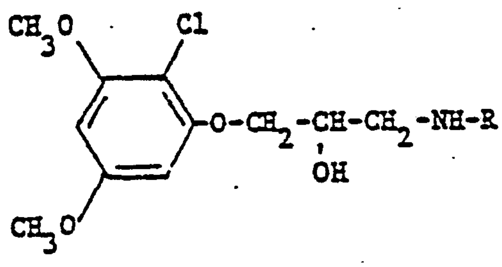

(I)

worin R für $CH(CH_3)_2$ oder $C(CH_3)_3$ steht; und
(ii) ihren Additionssalzen.

2. 1-(2-Chlor-3,5-dimethoxyphenoxy)-3-tert.butylamino-2-propanol und seine Additionssalze.

3. 1-(2-Chlor-3,5-dimethoxyphenoxy)-3-isopropylamino-2-propanol und seine Additionssalze.

4. Therapeutische Zusammensetzung, dadurch gekennzeichnet, daß sie in Verbindung mit einem physiologisch akzeptablen Exzipienten mindestens ein 3-Alkylamino-1-(2-chlor-3,5-dimethoxyphenoxy)-2-propanol der Formel (I) nach Anspruch 1 oder eines seiner nichttoxischen Additionssalze enthält.

5. Verfahren zur Herstellung einer Verbindung der Formel (I) nach Anspruch 1, dadurch gekennzeichnet, daß man a) ein 2-Chlor-3,5-dimethoxyphenol der Formel

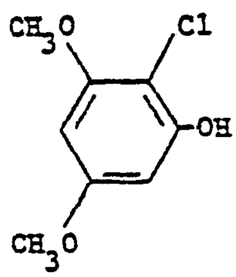

(II)

mit dem Epichlorhydrin III in Gegenwart von Pyridin zur Erzielung von 1-(2-Chlor-3,5-dimethoxyphenoxy)-2,3-epoxypropan der Formel

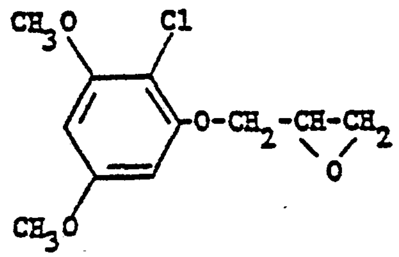

(IV)

zur Umsetzung bringt und danach

b) das so erhaltene Epoxid IV mit einem Amin

$NH_2R$ (V)

(worin R für $i\text{-}C_3H_7$ oder $t\text{-}C_4H_9$ steht) in einem $C_1$-$C_3$-Niedrigalkohol zur Umsetzung bringt.

6. Verfahren nach Anspruch 5, dadurch gekennzeichnet, daß man in Stufe a) 1 Mol von II mit 4 bis 10 Mol von III während 2 bis 5 h bei einer Temperatur zwischen 100 und 120° C zur Umsetzung bringt, und daß man in Stufe b) 1 Mol des Epoxids IV mit 8 bis 12 Mol des Amins V in Gegenwart von Äthanol bei der Rückflußtemperatur des Reaktionsmediums während mindestens 0,5 h zur Umsetzung bringt.

**Patentansprüche** für den Vertragsstaat AT:

1. Verwendung eines 3-Amino-1-phenoxy-2-propanol-Derivats, ausgewählt aus der Gruppe bestehend aus

(i) den 3-Alkylamino-1-(2-chlor-3,5-dimethoxyphenoxy)-2-propanolen der allgemeinen Formel

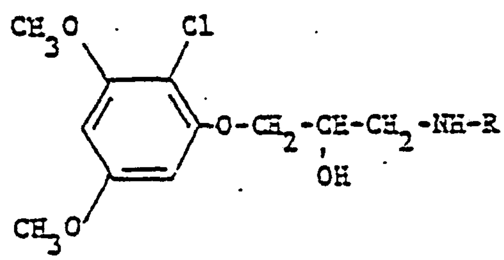

(I)

worin R für $CH(CH_3)_2$ oder $C(CH_3)_3$ steht; und

(ii) ihren nichttoxischen Additionssalzen.

zur Herstellung eines Medikaments.

2. Verwendung nach Anspruch 1, dadurch gekennzeichnet, daß die Verbindung 1-(2-Chlor-3,5-dimethoxyphenoxy)-3-tert. butylamino-2-propanol und seine Additionssalze ist.

3. Verwendung nach Anspruch 1, dadurch gekennzeichnet, daß die Verbindung 1-(2-Chlor-3,5-dimethoxyphenoxy)-3-isopropylamino-2-propanol und seine Additionssalze ist.

4. Verwendung nach einem der Ansprüche 1 bis 3, dadurch gekennzeichnet, daß mindestens ein 3-Amino-1-phenoxy-2-propanol-Derivat oder eines seiner nichttoxischen Additionssalze in Verbindung mit einem physiologisch akzeptablen Exzipienten vorliegt.

5. Verfahren zur Herstellung einer Verbindung der Formel (I) nach Anspruch 1, dadurch gekennzeichnet, daß man a) ein 2-Chlor-3,5-dimethoxyphenol der Formel

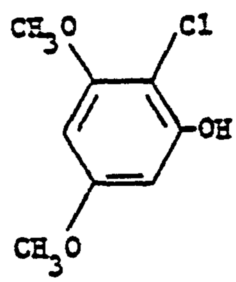

(II)

mit dem Epichlorhydrin III in Gegenwart von Pyridin zur Erzielung von 1-(2-Chlor-3,5-dimethoxyphenoxy)-2,3-epoxypropan der Formel

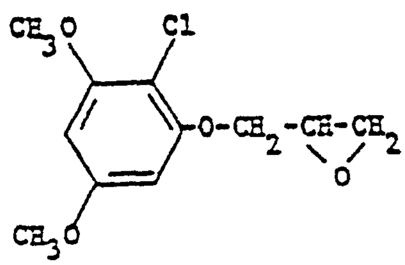

(IV)

zur Umsetzung bringt und danach

b) das so erhaltene Epoxid IV mit einem Amin

$NH_2R$ (V)

(worin R für i-$C_3H_7$ oder t-$C_4H_9$ steht) in einem $C_1$-$C_3$-Niedrigalkohol zur Umsetzung bringt.

6. Verfahren nach Anspruch 5, dadurch gekennzeichnet, daß man in Stufe a) 1 Mol von II mit 4 bis 10 Mol von III während 2 bis 5 h bei einer Temperatur zwischen 100 und 120° C zur Umsetzung bringt, und daß man in Stufe b) 1 Mol des Epoxids IV mit 8 bis 12 Mol des Amins V in Gegenwart von Äthanol bei der Rückflußtemperatur des Reaktionsmediums während mindestens 0,5 h zur Umsetzung bringt.

**Claims** for the contracting states: BE, CH, DE, FR, GB, IT, LI, LU, NL, SE:

1. 3-amino-1-phenoxy-2-propanol derivative, characterized in that it is selected from the group constituted by (i) the 3-alkylamino-1-(2-chloro-3,5-dimethoxyphenoxy)-2-propanols responding to the general formula

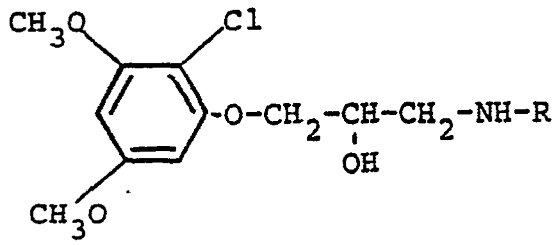

(I)

in which R is $CH(CH_3)_2$ or $C(CH_3)_3$; and

(ii) their addition salts.

2. 1-(2-chloro-3,5-dimethoxyphenoxy)-3-tertiobutylamino-2-propanol and its addition salts.

3. 1-(2-chloro-3,5-dimethoxyphenoxy)-3-isopropylamino-2-propanol and its addition salts.

4. Therapeutical composition, characterized in that it contains, in association with a physiologically acceptable excipient, at least one 3-al-kylamino-1-(2-chloro-3,5-dimethoxyphenoxy)-2-propanol of formula (I) according to claim 1 or one of its non toxic addition salts.

5. Process for preparing a compound of formula (I) according to claim 1, characterized in that

a) a 2-chloro-3,5-dimethoxyphenol of formula

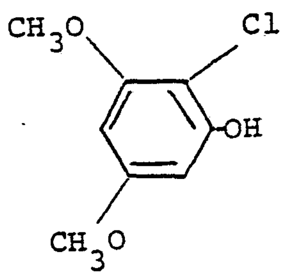

(II)

is reacted with the epichlorohydrin III, in the presence of pyridine to obtain the 1-(2-chloro-3,5-dimethoxyphenoxy)-2,3-epoxypropane of formula

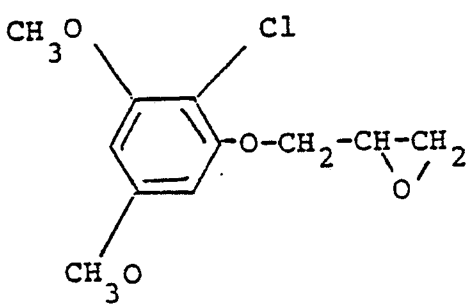

(IV)

then

b) the epoxy IV thus obtained is reacted with an amine

$NH_2R$ (V)

(wherein R is $i-C_3H_7$ or $t-C_4H_9$) in a lower alcohol in $C_1$-$C_3$.

6. Process according to claim 5, characterized in that, during step a) 1 mol of II is reacted with 4 to 10 mols of III for 2 to 5 hours at a temperature of between 100 and 120°C; and in that, during step b) I mol of epoxyde IV is

reacted with 8 to 12 mols of amine V in the presence of ethanol at the reflux temperature of the reaction medium for at least half an hour.

**Claims** for the contracting state: AT

1. use of a 3-amino-1-phenoxy-2-propanol derivative, selected from the group constituted by
(i) the 3-alkylamino-I-(2-chloro-3,5-dime-thoxyphenoxy)-2-propanols responding to the general formula:

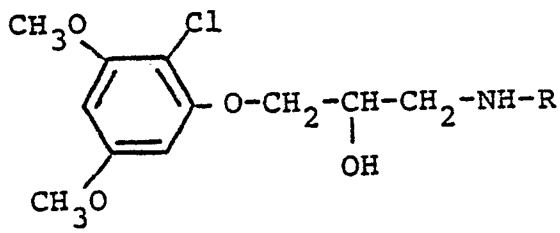

(I)

in which R is $CH(CH_3)_2$ or $C(CH_3)_3$; and
(ii) their non toxic addition salts for manufacturing a drug.

2. Use according to claim 1, characterized in that the compound is 1-(2-chloro-3,5-dimetho-xyphenoxy)-3-tertiobutylamino-2-propanol and its addition salts.

3. Use according to claim 1, characterized in that the compound is 1-(2-chloro-3,5-dimetho-xyphenoxy)-3-isopropyl-amino-2-propanol and its addition salts.

4. Use according to any one of claims 1 to 3, characterized in that at least one derivative of 3-amino-1-phenoxy-2-propanol or one of its non toxic addition salts is in association with a physiologically acceptable excipient.

5. Process for preparing a compound of formula (I) according to claim 1, characterized in that
a) a 2-chloro-3,5-dimethoxyphenol of formula

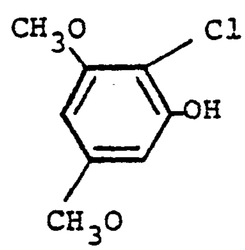

(II)

is reacted with the epichlorohydrin III, in the presence of pyridine to obtain the 1-(2-chloro-3,5-dimethoxyphenoxy)-2,3-epoxypropane of formula

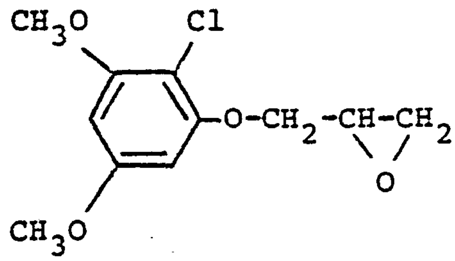

(IV)

then

b)the epoxy IV thus obtained is reacted with an amine

$NH_2R$ (V)

(wherein R is $i-C_3H_7$ or $t-C_4H_9$) in a lower alcohol in $C_1-C_3$.

6. Process according to claim 5, characterized in that, during step a), 1 mol of II is reacted with 4 to 10 mols of III for 2 to 5 hours at a temperature of between 100 and 120° C; and in that, during step b), 1 mol of epoxy IV is reacted with 8 to 12 mols of amine V in the presence of ethanol at the reflux temperature of the reaction medium for at least half an hour.